# EUROPEAN PATENT SPECIFICATION

(11) **EP 3 194 357 B1**
(45) Date of publication and mention of the grant of the patent: **08.05.2019**
(21) Application number: 14853146.0
(22) Date of filing: 19.09.2014
(51) Int. Cl.: C07C 67/08, C07C 69/82, C07C 69/28, C07C 69/54

(54) **PROCESS FOR PREPARING CARBOXYLIC ACID ESTERS IN THE PRESENCE OF A TITANIUM-CONTAINING CATALYST**
VERFAHREN ZUR HERSTELLUNG VON CARBONSÄUREESTERN IN ANWESENHEIT EINES TITANHALTIGEN KATALYSATORS
PROCÉDÉ DE PRÉPARATION D'ESTERS D'ACIDES CARBOXYLIQUES EN PRÉSENCE D'UN CATALYSEUR CONTENANT DU TITANE

(43) Date of publication of application: 26.07.2017
(73) Proprietor: Public Joint Stock Company "Sibur Holding", Tyumen region 626150 (RU)
(72) Inventor: KOLESNIK, Vasily Dmitrievich, Tomsk 634021 (RU); NOSIKOV, Alexei Alexandrovich, Tomsky raion s. Turuntaevo 634534 (RU); KALININ, Rodion Georgievich, Krasnoyarsk 660130 (RU); BOBRIKOVA, Anastasia Alexandrovna, Tomsk 634031 (RU); SHERSTOBITOV, Ivan Anatolievich, Anzhero-Sudzhensk 652480 (RU)
(74) Representative: Zacco Denmark A/S
(86) International application number: PCT/RU2014/000700
(87) International publication number: WO 2016/043616

(56) References cited:
- US-A- 2 727 881
- US-A- 3 056 818
- US-A- 3 418 359

## Description

The invention relates to the field of organic synthesis, in particular, to a process for preparing esters by esterification of carboxylic acids in the presence of a catalyst based on titanium organic compounds.

Carboxylic acid esters are widely used in industry. They are used as solvents, plasticizers, flavoring agents, etc. For example, dimethyl esters of terephthalic acid are used for the manufacture of polyester fibers, filaments, enamels and films. Dioctyl esters of terephthalic acid are used as plasticizers for various polymer materials, in particular, in the manufacture of polyvinyl chloride. Such an ester as ethyl acrylate is used as a comonomer in copolymerization reactions with other monomers. Acrylic acid esters, in particular methyl acrylate, are used for the manufacture of plastic materials, transparent films of high mechanical strength. Acrylic ester resins are used also in the paint industry.

### BACKGROUND

Methods of esterification of carboxylic acids with alcohols in the presence of various catalysts are known for a long time. Mineral acids were used as catalysts. However, the use of such catalysts led to corrosion of equipment and made neutralization of the catalyst after reaction more expensive.

The use of organic acids in the esterification reaction, in particular alkyl and aryl sulfonic acids, allowed the reduction of said problems.

The publication of International application WO 2012/026661 discloses a process for preparing an alkyl (meth)acrylates by esterification of an acid with an alcohol and in the presence of acids used as a catalyst, in particular, alkyl and aryl sulfonic acids. Esterification at a temperature of more than 100-120°C leads to a reduction in the selectivity of the esterification process and to the formation of a considerable amount of by-products, thus requiring additional treatment steps to increase the total yield of a product.

The problem concerning corrosion and an increase in the catalyst activity was addressed by using a solid-phase catalyst such as solid superacidic catalysts based on titanium and zirconium compounds ("Solid catalysts treated with anions. 13. Synthesis of esters from terephthalic and phthalic acids with n-octyl and 2-ethylhexyl alcohol, acrylic acid with ethanol and salicylic acid with methanol catalyzed by solid superacid", Applied Catalysis, 1985,18(2), 401-4 (English)) or cation exchange resins (EP 1726579, US 20110190464).

The use of homogenous titanium-organic catalysts in esterification processes made it possible to broaden significantly the spectrum of obtainable esters widely used in industrial scales.

Patent application JP 60004151 A discloses a process for preparing terephthalic acid esters by esterification of terephthalic acid with alcohols, for example 2-ethylhexanol, in the presence of Ti(OPr-iso)₄. The process conducted under nitrogen atmosphere and pressure of up to 1.0 kg/cm² for 30 min at 220°C, or under pressure of 3.5 kg/cm² for 3 hours at 220°C provides a conversion rate of 91%.

The publication of international application WO 2007021475 discloses production of di(2-ethylhexyl)terephthalate by esterification of terephthalic acid with 2-ethylhexanol in the presence of titanium alkoxides comprising from 1 to 18 carbon atoms in the alkyl group, in particular titanium tetraisopropoxide, used as a catalyst.

Application KR 2009092067 discloses a reaction of aliphatic and aromatic acids or anhydrides thereof with alcohols in the presence of titanium catalysts such as tetraoctyl or tetrabutyl titanates, wherein a solvent further comprises a neutralizing agent and a neutralizing-adsorbing agent, followed by distillation and filtration of the mixture. Aromatic acids used in the process also include dicarboxylic acids.

Patent US 3418359 discloses esterification in the presence of a two-component catalyst comprising a titanate of general formula Ti(OR)₄, wherein R is an acyl group, an unsaturated hydrocarbon group, a cyclohexyl group, or a phenyl group, together with zinc dicarboxylate. The process can be used for esterification of aromatic and aliphatic acids. However, regardless of the indication of aliphatic acids, there are absent examples of a particular process of esterification with them in the presence of titanium catalysts, that would be characterized by the process parameters and demonstrate the obtained results.

The process closest to the claimed one is a process of esterification of carboxylic acids or anhydrides thereof with aliphatic primary or secondary alcohols in liquid phase in the presence of a titanium catalyst, as disclosed in patent US 3056818. A catalyst used in this process is a compound of generic formula MX, wherein M is titanium or zirconium, X is selected from the group consisting of hydroxyl, alkoxy, acyloxy, and aminoalkoxy groups and halogen atoms having atomic weights from 35.457 to 79.916, and wherein at least one X is an organic radical having from 2 to 18 carbon atoms. The catalyst is tetraisopropyl titanate or titanium tetraphenolate. The amount of titanium tetraphenolate used in this patent (Example VI) is 5300 ppm. Based on the results obtained in examples of particular embodiments of the invention, it can be concluded that titanium (IV) tetraryl complexes used in the present invention have no advantages in their activity over titanium isopropoxide. In addition, the process requires a large amount of a catalyst.

A disadvantage of all aforesaid methods is a long reaction time and a large amount of a catalyst to be used. In addition, a complete conversion of an acid to a product is not achieved. A consequence of this drawback is a low performance of an apparatus for the manufacture of products.

Thus, there is a need to develop a process of esterification of carboxylic acids with alcohols in the presence of a catalyst used in a significantly less amount, which methods allow the reaction with a good conversion rate and a high yield of a target product over a shorter period of time.

The objective of the invention is to develop an improved process for preparing carboxylic acid esters.

The objective is addressed by the use of catalysts in the esterification reaction, as described in the present invention, wherein the catalysts are catalysts based on titanium organic compounds comprising at least two alkoxy groups and two aryloxy groups, which compounds, as the present inventors has unexpectedly found, have a higher catalytic activity and an increased stability under the process conditions, compared with the earlier known catalysts, thus allowing the reaction esterification to continue until the conversion is completed. The claimed process makes it possible to reduce the amount of a used catalyst and the time of the process duration while increasing the conversion rate of initial reagents and the yield of a target product.

### SUMMARY OF THE INVENTION

The present invention relates to a process for preparing carboxylic acid esters, comprising esterification of a carboxylic acid with an alcohol in the presence of a titanium-containing catalyst selected from compounds of a general formula:

Tiₙ(OR)ₓ(OR')ₓO_{y},

wherein
n is an integer from 1 to 4;
y is an integer from 0 to 6;
x can be the same or different and is an integer from 2 to 8;
R is a linear or branched C₁-C₁₈alkyl, C₃-C₁₈cycloalkyl; R' is aryl optionally comprising an electron-donor substituent;
or a mixture thereof,
with the proviso that
if n is 1, then x is 2 and y is 0; and
if n>1, then the compounds comprise at least two alkoxy groups and two aryloxy groups.

The present invention also relates to a process for preparing carboxylic acid esters, comprising esterification of a carboxylic acid with an alcohol in the presence of a titanium-containing catalyst selected from compounds of general formula I or II: 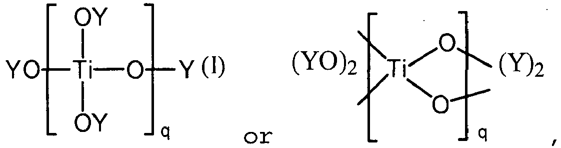 wherein
q is an integer from 1 to 4;
Y is independently R or R'
R is a linear or branched C₁-C₁₈alkyl, C₃-C₁₈cycloalkyl; R' is aryl optionally comprising an electron-donor substituent;
or a mixture thereof with the proviso that the compounds comprise at least two alkoxy groups and two aryloxy groups.

### DESCRIPTION OF THE DRAWINGS

Fig.1 represents the ¹H NMR spectrum of the catalyst prepared according to Example la.
Fig.2 represents the ¹³C NMR spectrum of the catalyst prepared according to Example la.
Fig.3 represents the ¹H NMR spectrum of the catalyst prepared according to Example 1b.
Fig.4 represents the ¹³CNMR spectrum of the catalyst prepared according to Example 1b.
Fig.5 represents the ¹H NMR spectrum of the catalyst prepared according to Example lc.

### DETAILED DESCRIPTION OF THE INVENTION

C₁-C₁₈ as used in the present invention can preferably be methyl, ethyl, n-propyl, isopropyl, n-butyl, *sec*-butyl, isobutyl, *tert*-butyl, n-pentyl, *sec*-pentyl, isopentyl, *tert*-pentyl, neopentyl, n-hexyl, isohexyl, neohexyl, *sec-*hexyl, and *tert*-hexyl.

C₃-C₁₈cycloalkyl as used in the present invention is preferably C₅-C₇cycloalkyl, more preferably cyclopentyl, cyclohexyl, methycyclopentyl, methylcyclohexyl, dimethylcyclopentyl, ethylcyclopentyl, and cycloheptyl.

The electron-donor substituent of the aryl group is preferably selected from C₁-C₆alkyl, aryl, C₁-C₆alkoxy, C₁-C₆alkylamino, and C₁-C₆alkylthio.

The alkyl part in the electron-donor substituent as used in the present invention is preferably methyl, ethyl, n-propyl, isopropyl, n-butyl, *sec*-butyl, isobutyl, *tert-*butyl, n-pentyl, *sec*-pentyl, isopentyl, *tert*-pentyl, 2,2-dimethylpentyl, n-hexyl, isohexyl, 2,2-dimethylhexyl, *sec-*hexyl, and tert-hexyl.

Aryl used in the present invention is preferably phenyl.

The numbers of alkoxy groups and aryloxy groups in the catalyst molecule can be the same or different and can be from 2 to 8 groups.

In a preferable variant, the numbers of alkoxy groups and aryloxy groups in the catalyst molecule are the same and are from 2 to 5 groups.

The numbers of alkoxy groups and aryloxy groups in the catalyst molecule also can be different and can be independently 2, 3, 4, 5, 6, 7, and 8 groups depending on the number of titanium and oxygen atoms in the molecule.

The maximum number of aryloxy and alkoxy groups in the catalyst molecule with one titanium atom can be 4, with two titanium atoms can be 6, with three titanium atoms can be 8, and with four titanium atoms can be 10.

The catalyst according to the invention can be used as an individual compound and in the form of a mixture of compounds.

The catalyst according to the present invention can exist in different structural forms, in particular, in mono-, di-, tri- or tetrameric forms.

Preferred catalysts used in the present invention are catalysts having the following structures:

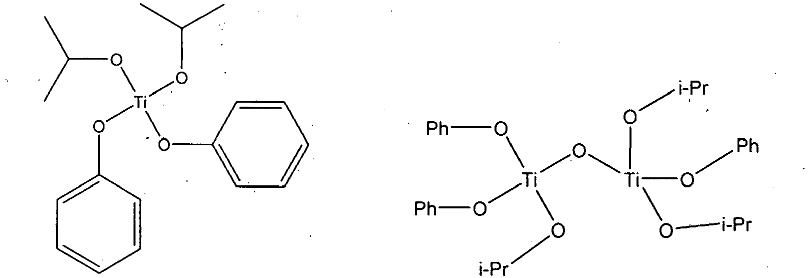

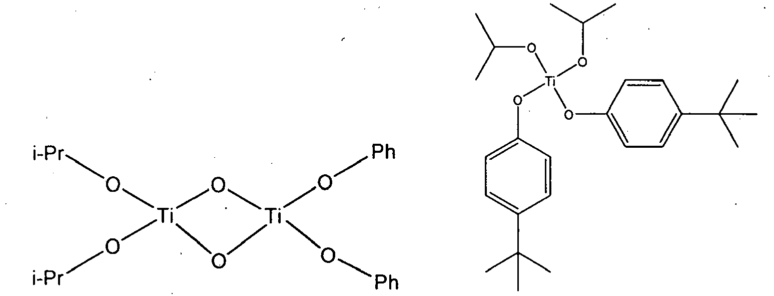

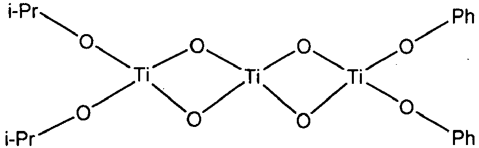

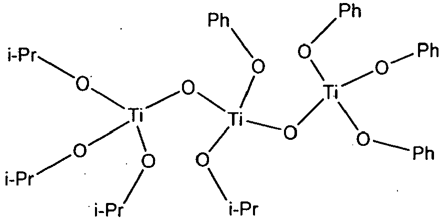

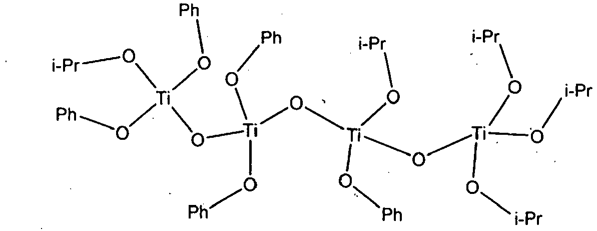

The catalysts according to the present invention are prepared from chemically pure initial compounds such as phenol or its derivatives and tetraalkyl titanates used in an organic solvent, preferably dehydrate toluene, in an inert gas, preferably nitrogen, by adding tetraalkyl titanate to the solution of a hydroxyaromatic compound, preferably phenol or a derivative thereof, in an organic solvent under stirring and at an elevated temperature, preferably ranging between 60 and 90°C.

The reaction time is several hours, preferably from 10 to 12 hours, followed, if necessary, by removal of the solvent under reduced pressure.

Titanates are known to be an octahedral coordination polyhedron consisting of six ligands, wherein two out of said six ligands are bound to the titanium atom by a coordination bond, and the rest of them are bound by a covalent bond.

More basic ligands (alkoxides) are easily substituted with less basic ones (aryloxides, carboxilates). Titanates thereby formed are more stable and in a less hydrolysable. However, in esterification reactions, the catalytic activity of a compound, which ligand environment fully consists of phenoxy radicals, is reduced due to the bond Ti-O-Ar that is chemically more inertial.

A high content of alkoxy ligands also can reduce the activity because of decreasing the concentration of active centers, which is due to oligomerization reactions caused by a trace amount of water.

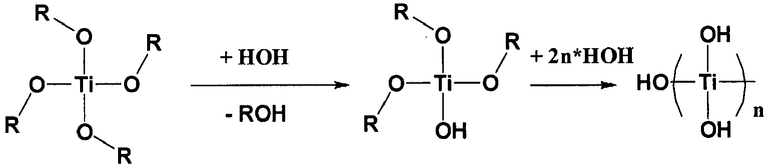

Based on the foregoing, it would have been expected that compounds of formula I would have the disadvantages which are the same for compounds having either only alkoxy or only aryloxy ligands. However, the present inventors have unexpectedly found that a compound comprising both types of ligands in an equal amount is characterized by significantly higher activity compared with tetraaryl titanates, and an improved stability compared with tetraalkyl titanates, which results in higher efficiency of such a compound (product yield per catalyst unit) in esterification reactions.

In the presence of said catalyst, various carboxylic acids and alcohols are used as initial compounds in the esterification reaction.

Acids, used herein, are mono- and polybasic carboxylic acids having a linear, branched or cyclic structure, including unsaturated, aromatic and aliphatic structures. Carboxylic acids comprising from 3 to 18 carbon atoms are preferred. It is more preferable to use in the process according to the invention 2-ethylhexanoic, terephthalic, isophthalic, phthalic, benzoic, acrylic, metacrylic, stearic, adipic, succinic, sebacic, crotonic, cinnamonic, oleinic, linolic, linolenic, maleic, fumaric, palmitic, lauric acid, and palmitoleic acids.

Alcohols used therein are mono- and polyalcohols of a linear or branched structure, including alcohols comprising one or more heteroatoms such as O, S or N. Alcohols comprising from 1 to 18 carbon atoms are preferred. It is preferable to use in the process according to the invention alcohols comprising from 1 to 8 carbon atoms, wherein alcohols comprising from 4 to 8 carbon atoms, such as n-butanol, sec-butanol, isobutanol, n-amyl alcohol, sec-amyl alcohol, isoamyl alcohol, n-octyl alcohol, 2-ethylhexyl alcohol, monoethylene glycol, diethylene glycol, triethylene glycol, and tetraethylene glycol, are more preferred.

The esterification reaction is conducted at a carboxylic/hydroxyl group ratio in the total flow of raw materials of from 5:1 to 1:5, preferably from 2:1 to 1:2, more preferably from 1.3:1 to 1:1.3. A reagent that is more effective in the formation of an azeotropic mixture with water is taken in excess. Beyond these ratios, the process is not practically feasible since too large excess of one of the initial reagents leads to the necessity of separation of a large amount of unreacted reagent from the final product - an ester, which is economically unreasonable.

The process according to the invention comprises reacting alcohols with carboxylic acids in the presence of a titanium-containing catalyst under stirring for a required time at elevated temperature, followed by separation of a target ester from the reaction products.

Since the esterification reaction is an equilibrium reaction, in order to additionally increase the reagent conversion rate and the ester yield, water that formed in the reaction is distilled off as azeotrope and then separated. An access amount of the reagent, which is more effective in the formation of an azeotropic mixture with water, is taken depending on the structure of the used acid and alcohol. The separated alcohol or acid is recycled to the reactor. The azeotrope distillation rate can be regulated, in particular by bubbling the reaction mixture with nitrogen.

The process is conducted at a temperature ranging between 100 and 300°C, preferably between 160 and 250°C, more preferably between 175 and 220°C, and under pressure that provides boiling the reaction mixture at said temperature with distillation of water azeotrope until the formation of water ceases. The catalyst activity is reduced at a temperature lower than 100°C, but at a temperature of higher then 300°C the yield of by-products increases and, correspondingly, the yield of the target product decreases.

The catalyst can be loaded to a reactor with initial compounds or after achieving a preferable temperature of the reaction. The amount of the loaded catalyst depends on the amount of titanium comprised therein and is from 50 to 5000 ppm per weight of the reaction mixture, preferably from 200 to 1000 ppm of the reaction mixture, more preferably from 500 to 700 ppm per weight of the reaction mixture. The use of the catalyst in more amounts is not feasible in economic terms.

The process for preparing esters can be conducted in both batch and continuous esterification reactors, wherein any prior art-known device suitable for the esterification reaction can be used. The reactor should provide the reaction under nitrogen atmosphere at continuous distillation of a heteroazeotropic mixture to be further separated. The azeotrope distillation rate is adjusted by an throttle valve to not more than a certain level that ranges between 0 and 6 reactor volumes per hour, preferably between 0.2 and 2 reactor volumes per hour. The rate of feeding nitrogen to the reactor is from 0 to 10 reactor volumes per hour, preferably from 0.1 to 4 reactor volumes per hour.

Compared with processes using esterification catalysts known from the prior art, the process according to the present invention provides an increased yield of the product per mole of the catalyst and, as consequence, an increase in the efficiency of the equipment. For example, the use of a new titanium organic catalyst provides at least additional 12 kg/hour DOTP (dioctylterephthalate) per m³ of reaction volume, that gives further from 96 tons DOTP per 1 M3 reaction volume per year, avoiding additional energy consumption. Carboxylic esters are separated from the reaction products by the removal of unreacted acid and/or alcohol by methods known from the prior art that comprise removing acid impurities by neutralization thereof, followed, if necessary, by filtration (in case of solid acids, for example terephthalic acid), and/or distilling unreacted alcohol by direct steam or vacuum distillation or other known methods, followed, if necessary, by separation of the reaction product by rectification.

When initial comounds, for example unsaturated acids, and/or the product of the esterification reaction are compounds tending to polymerization, the esterification process is conducted in the presence of a polymerization inhibitor. The polymerization inhibitor used in the present invention can be any suitable inhibitor known from the prior art, in particular, a phenol type inhibitors. Hydroquinone and p-methoxyphenol are preferred as inhibitor. The quantity of the polymerization inhibitor used in the present invention is 0.01 to 1%, preferably 0.05 to 0.2%, and more preferably 0.1 to 0.15% by weight of the reaction mixture.

The present invention is illustrated by the following examples.

### EXAMPLES

### Preparing an esterification catalyst

Example 1a. The initial reagents, in particular phenol and titanium tetraisopropoxide, are purified by vacuum distillation.

Phenol (7.04 mmol) and dehydrated toluene (10 ml) are loaded to a 25 ml two-necked flask equipped with a stirrer and filled with dry nitrogen. Titanium tetraisopropoxide (3.52 mmol) is added to the obtained solution in a single portion. The obtained solution is stirred at 80°C for 12 hours under an inert gas atmosphere.

Toluene is removed in a rotary evaporator at 80°C and under pressure of 13.221 to 0.267 kPa (100 to 2 mm Hg). The obtained resin-like substance has a red-orange color.

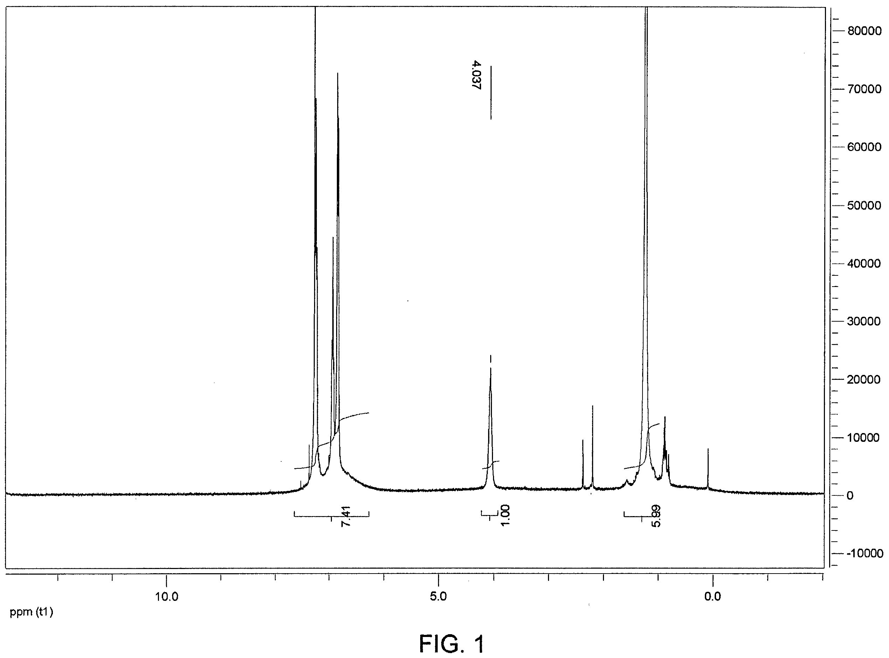

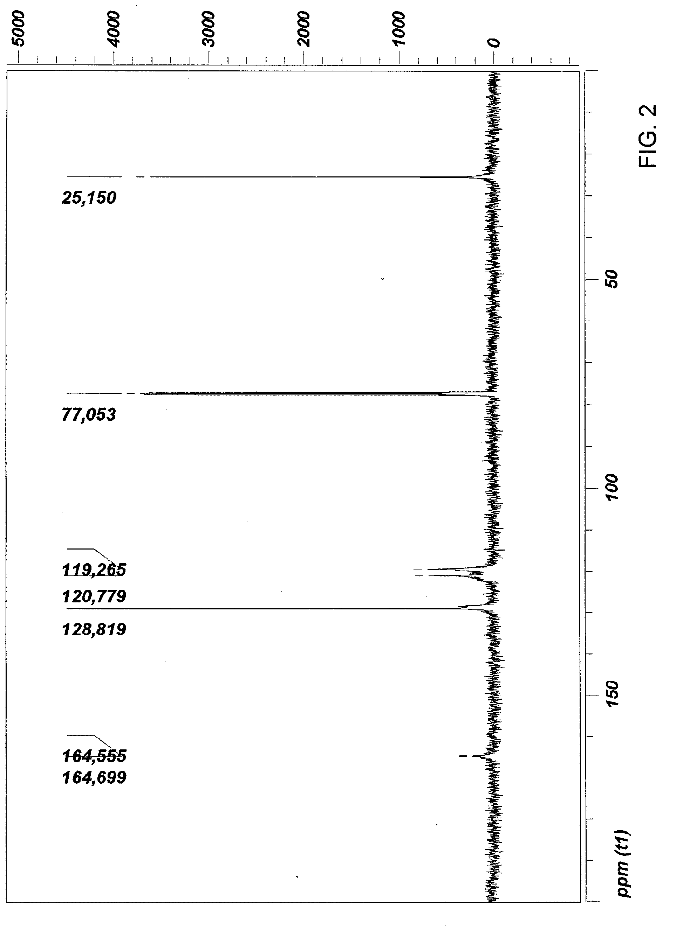

The ¹H NMR and ¹³C NMR spectra of the obtained compound are shown in Figs 1 and 2.

Example 1b. p-*tert*-Butylphenol (7.04 mmol) and dehydrate toluene (10 ml) are loaded to a 25 ml two-necked flask equipped with a stirrer and filled with dry nitrogen. Titanium tetraisopropoxide (3.52 mol) is added to the obtained solution in a single portion. The obtained solution is stirred at 80 °C for 12 hours under an inert gas atmosphere.

Toluene is removed in a rotary evaporator at 80°C and under pressure of 13.332 to 0.267 kPa (100 to 2 mm Hg). The obtained resin-like substance has a red-orange color.

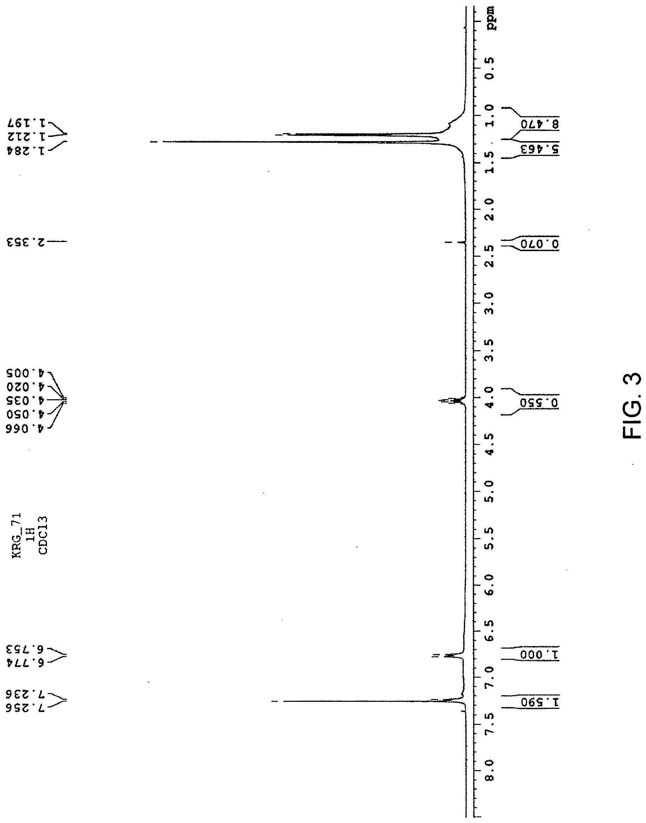

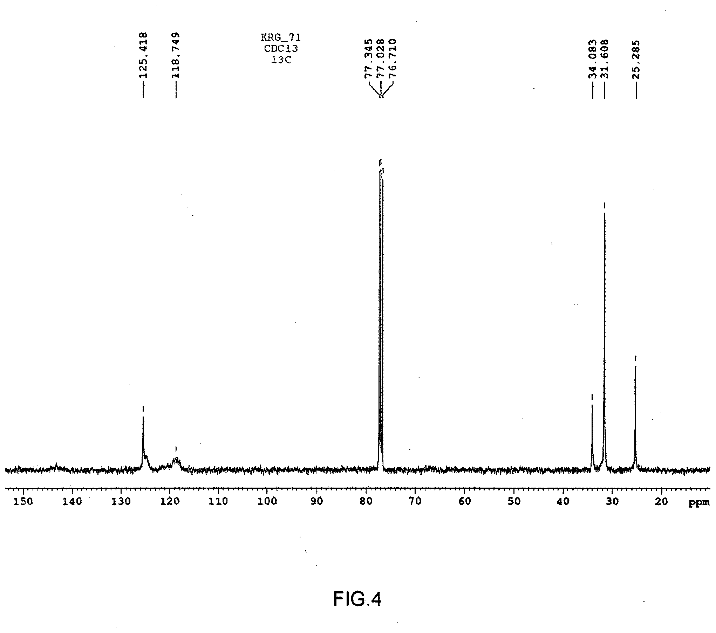

The ¹H NMR and ¹³C NMR spectra of the obtained compound are shown in Figs 3 and 4.

Example 1c. p-Nitrophenol (7.04 mmol) and dehydrate toluene (10 ml) are loaded to a 25 ml two-necked flask equipped with a stirrer and filled with dry nitrogen. Titanium tetraisopropoxide (3.52 mmol) is added to the obtained solution in a single portion. The obtained solution is stirred at 80°C for 12 hours under an inert gas.

Toluene is removed in a rotary evaporator at 80°C and under pressure of 13.332 to 0.267 kPa (100 to 2 mm Hg). The obtained crystalizing compound has a yellow color.

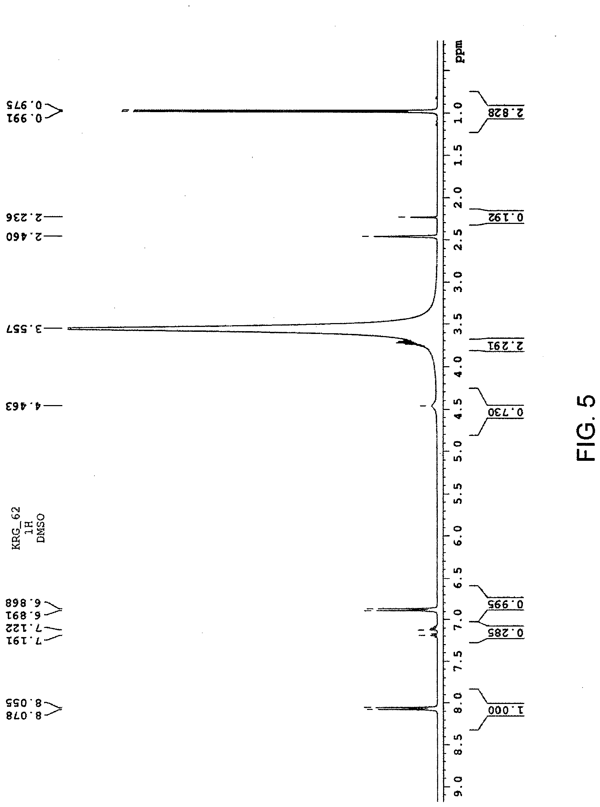

The 1H NMR spectra of the obtained compound are shown in Fig.5.

### Examples of an esterification process

### Example 2 (comparative). Preparing 2-ethylhexyl terephthalate (DOTP) in the presence of titanium tetraisopropoxide

Terephthalic acid (TPA) (0.63 mol) and 2-ethylhexanol (1.44 mol) at a molar ratio of 1:2.3 are loaded to a 500 ml steel reaction vessel equipped with a heatable jacket, a steam control valve, and a Dean-Stark trap for distillation and separation of a water-alcohol azeotropic mixture. The mixture is heated to 200°C and then titanium tetraisopropoxide is added in an amount of 600 ppm per acid-alcohol mixture. The reaction is continued for 4 hours under stirring at a rate of 800 rpm, while passing through nitrogen gas at a rate of 0.05 L/min. During the reaction, the water-alcohol azeotropic mixture is distilled. The distilled alcohol is recycled into the reaction mixture. When the reaction is completed, the mixture is cooled and unreacted acid is filtered off. The yield of the reaction and the calculated efficiency of the process are given in Table 1.

### Example 3 (comparative). Preparing 2-ethylhexylterephthalate in the presence of a titanium tetraphenoxide catalyst

The process is carried out as described in Example 2. The yield of the reaction and the calculated efficiency of the process are given in Table 1.

### Example 4. Preparing 2-ethylhexylterephthalate in the presence of the catalyst prepared as disclosed in Example 1a

The process is carried out as described in Example 2. The yield of the reaction and the calculated efficiency of the process are given in Table 1.

### Example 5. Preparing 2-ethylhexylterephthalate in the presence of the catalyst prepared as disclosed in Example 1b

The process is carried out as described in Example 2. The yield of the reaction and the calculated efficiency of the process are given in Table 1.

### Example 6 (comparative). Preparing 2-ethylhexylterephthalate in the presence of the catalyst prepared as disclosed in Example 1c

The process is carried out as described in Example 2. The yield of the reaction and the calculated efficiency of the process are given in Table 1.

**Table 1. Results of the synthesis of DOTP**

| Example No. | Catalyst | DOTP yield, % | Efficiency, kg [DOTP] /m³*h |
|---|---|---|---|
| 2 (Comparative) | Titanium tetraisopropoxide | 90.0 | 110.83 |
| 3 (Comparative) | Titanium tetraphenoxide | 96.2 | 118.60 |
| 4 | prepared as described in Example 1a | 99.5 | 122.84 |
| 5 | prepared as described in Example 1b | 94.8 | 116.96 |
| 6 | prepared as described in Example 1c | 90.1 | 111.20 |

Data in Table 1 clearly show that the use of catalyst comprising both aryloxy and alkoxy groups unexpectedly allows to increase the product yield and the efficiency of the process at the same amount of a used catalyst. In addition, the obtained data also show that the presence of an electron-donor substituted aryl group in the catalyst structure makes it more effective in comparison with the similar catalyst comprising an electron--withdrawing substituted aryl group.

### Example 7 (comparative). Preparing 2-ethylhexylacrylate in the presence of a titanium tetraisopropoxide catalyst

Acrylic acid (0.5 mol) and 2-ethylhexanol (0.69 mol) at a molar ratio of 1:1.38 are loaded under nitrogen atmosphere to a 250 ml three-necked flask equipped with a magnetic stirrer, an inert-gas bubbling system, a temperature gauge, and a Dean-Stark trap under reflux for distillation and separation of a water-alcohol azeotropic mixture. Hydroquinon (0.0012 mol) is added to the solution as an inhibitor. The receiver of the Dean-Stark trap is filled with 2-ethylhexanol. The reaction mixture is heated to 115°C and a titanium tetraisopropoxide catalyst is introduced in an amount of 600 ppm per acid-alcohol mixture in form of a 15% solution in 2-ethylhexanol. The reaction mixture is heated to 180°C, and the reaction is continued for 4 hours under continuous bubbling with nitrogen at a mixing rate of 600 rpm. During the reaction, the water-alcohol azeotropic mixture is distilled. The distilled alcohol is recycled to the reaction vessel. 2-Ethylhexylacrylate is separated from the reaction mixture by rectification. The acrylic acid conversion rate is 89%, and the selectivity of conversion of acrylic acid to 2-ethylhexylacrylate is 87%.

The yield of the ester obtained in the synthesis step is 77%.

### Example 8. Preparing 2-ethylhexylacrylate in the presence of the catalyst prepared as disclosed in Example 1a

The process is carried out as described in Example 7. The catalyst is used in an amount of 600 ppm per acid-alcohol mixture.

The acrylic acid conversion rate is 89%, and the selectivity of conversion of acrylic acid to 2-ethylhexylacrylate is 93%.

The yield of the ester formed in the synthesis step is 83%.

Data set forth in Examples 7 and 8 clearly show that the use of catalysts comprising both aryloxy and alkyloxy groups unexpectedly allows an increase in the selectivity and in the yield of the process.

### Example 9. Preparing an ester of 2-ethylhexanoic acid and triethylene glycol in the presence of the catalyst prepared as disclosed in Example 1a

Triethylene glycol (0.5 mol) and 2-ethylhexanoic acid (1.5 mol) at a molar ratio of 1:3 are loaded to a 500 ml two-necked flask equipped with a Dean-Stark trap for distillation and separation of a water-acid azeotropic mixture. The mixture is heated to 280°C, and then the catalyst is added in an amount of 600 ppm. The reaction is continued for 2 hours under stirring at a rate of 600 rpm. During the reaction, the water-acid azeotropic mixture is distilled. The distilled acid is recycled to the reaction vessel. When the reaction is completed, the product is separated by distillation under vacuum.

The obtained results are shown in Table 2.

### Example 10 (comparative) Preparing an ester of 2-ethylhexanoic acid and triethylene glycol in the presence of a titanium tetraphenoxide catalyst

The process is carried out as described in Example 9. Titanium tetraphenoxide is used as a catalyst in an amount of 800 ppm.

The obtained results are shown in Table 2.

### Example 11 (comparative) Preparing an ester of 2-ethylhexanoic acid and triethylene glycol in the presence of a titanium tetraisopropoxide catalyst

The process is carried out as described in Example 9. Titanium tetraisopropoxide is used as a catalyst in an amount of 800 ppm.

The obtained results are shown in Table 2.

**Table 2. Results of the synthesis of an ester of 2-ethylhexanoic acid and triethylene glycol**

| Example No. | Catalyst | Catalyst amount, ppm | Triethylene glycol conversion rate, % | Reaction time, h |
|---|---|---|---|---|
| 9 | prepared as described in Example 1a | 600 | 100.0 | 2 |
| 10 (Comparative) | Titanium tetraphenoxide | 800 | 100.0 | 3 |
| 11 (Comparative) | Titanium tetraisopropoxide | 800 | 100.0 | 2.5 |

Data in Examples 9-11 clearly show that the use of catalysts comprising both aryloxy and alkyloxy groups unexpectedly leads to a very high alcohol conversion rate at a significantly lower amount of a catalyst, and shorter process time.

## Claims

1. A process for preparing carboxylic acid esters, comprising esterification of a carboxylic acid with an alcohol in the presence of a titanium-containing catalyst selected from compounds of a general formula:
Tiₙ(OR)ₓ(OR')ₓO_{y}
wherein
n is an integer from 1 to 4;
y is an integer from 0 to 6;
x can be the same or different and is an integer from 2 to 8;
R is a linear or branched C₁-C₁₈alkyl, C₃-C₁₈cycloalkyl; R' is aryl optionally comprising an electron-donor substituent;
or a mixture thereof,
with the proviso that
if n is 1, then x is 2 and y is 0; and,
if n>1, then the compounds comprise at least two alkoxy groups and two aryloxy groups.

2. The process according to claim 1, wherein R' is phenyl optionally comprising an electron-donor substituent.

3. The process according to claim 2, wherein the electron-donor substituent is an C₁-C₆alkyl, aryl, C₁-C₆alkoxy, C₁-C₆dialkylamino, or C₁-C₆alkylthio group.

4. The process according to claim 3, wherein the alkyl part in the electron-donor substituent is methyl, ethyl, n-propyl, isopropyl, n-butyl, *sec*-butyl, isobutyl, *tert-*butyl, n-pentyl, *sec*-pentyl, isopentyl, *tert*-pentyl, 2,2-dimethylpropyl, n-hexyl, isohexyl, 2,2-dimethylbutyl, *sec-*hexyl, or *tert*-hexyl.

5. The process according to claim 3, wherein the aryl is phenyl.

6. The process according to 1, wherein R is C₁-C₆alkyl.

7. The process according to claim 6, wherein R is methyl, ethyl, n-propyl, isopropyl, n-butyl, *sec*-butyl, isobutyl, *tert*-butyl, n-pentyl, *sec*-pentyl, isopentyl, *tert*-pentyl, 2,2-dimethylpropyl, n-hexyl, isohexyl, 2,2-dimethylbutyl, *sec*-hexyl, or *tert*-hexyl.

8. The process according to claim 1, wherein x is the same and is an integer from 2 to 5.

9. The process according to claim 1, wherein the catalyst is a compound of general formula (I) or (II): 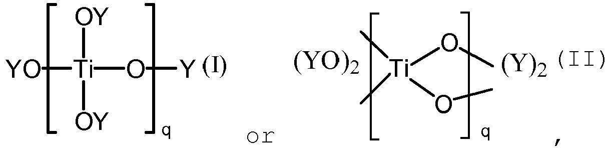 wherein
q is an integer from 1 to 4;
Y is independently R or R'; wherein R or R' is as defined in claim 1,
or a mixture thereof;
with the proviso that the compounds of general formula (I) and (II) comprise at least two alkyloxy and two aryloxy groups.

10. The process according to any one of claims 1 to 8, wherein the catalyst is a compound selected from:

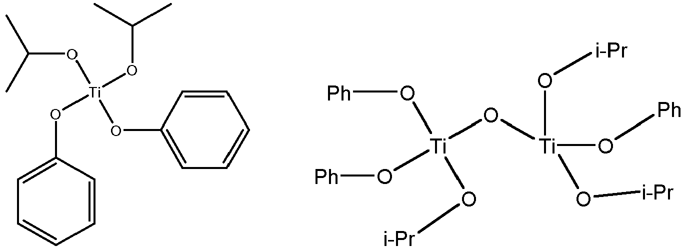

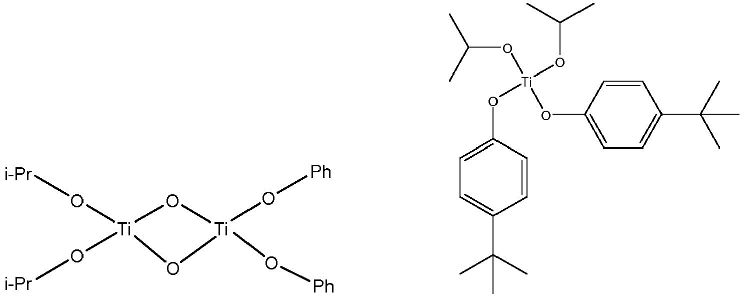

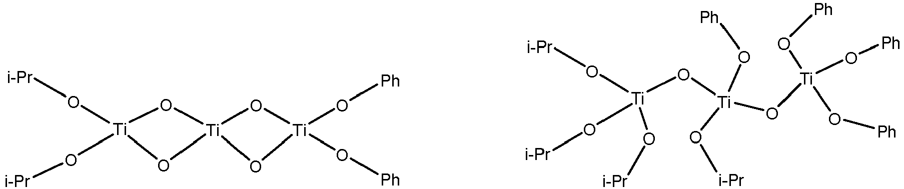

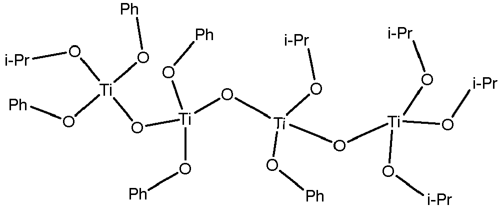

11. The process according to claim 9, wherein the catalyst is diisopropoxy diphenoxy titanium.

12. The process according to claim 9, wherein the catalyst is diisopropoxy di(p-*tert*-butyl)phenoxy titanium.

13. The process according to any one of claims 1 to 8, wherein the carboxylic acid is saturated or unsaturated mono- or polybasic carboxylic acid having a linear, branched or cyclic structure.

14. The process according to claim 12, wherein the carboxylic acid is an aromatic or aliphatic carboxylic acid.

15. The process according to claim 13, wherein the carboxylic acid comprises 3 to 18 carbon atoms.

16. The process according to claim 14, wherein the carboxylic acid is 2-ethylhexanoic, terephthalic, isophthalic, phthalic, benzoic, acrylic, metacrylic, stearic, adipic, succinic, sebacic, crotonic, cinnamonic, linolic, oleinic, linolenic, maleic, fumaric, palmitic, lauric acid, or palmitoleic acid.

17. The process according to any one of claims 1 to 8, wherein the alcohol is a monohydric or polyhydric alcohol having a linear or branched structure and optionally comprising one or more heteroatoms selected from O, S or N.

18. The process according to claim 17, wherein the alcohol comprises 1 to 18 carbon atoms.

19. The process according to claim 18, wherein the alcohol comprises 2 to 8 carbon atoms.

20. The process according to claim 19, wherein the alcohol is n-butanol, *sec*-butanol, isobutanol, n-amyl alcohol, *sec*-amyl alcohol, isoamyl alcohol, n-octanol, 2-ethylhexanol, monoethylene glycol, diethylene glycol, triethylene glycol, or tetraethylene glycol.

21. The process according to any one of claims 1 to 8, wherein the esterification is conducted at a carboxylic/hydroxyl group molar ratio of from 5:1 to 1:5, wherein a reagent forming an azeotropic mixture with water is taken in access.

22. The process according to claim 21, wherein the esterification is conducted at the carboxylic/hydroxyl group molar ratio of from 2:1 to 1:2.

23. The process according to claim 22, wherein the esterification is conducted at the carboxylic/hydroxyl group molar ratio of from 1.3:1 to 1:1.3.

24. The process according to any one of claims 1 to 8, wherein the esterification is conducted at a temperature ranging between 100 and 300°C and under pressure providing boiling the reaction mixture at said temperature.

25. The process according to claim 24, wherein the esterification is conducted at a temperature ranging between 160 and 250°C.

26. The process according to claim 25, wherein the esterification is conducted at a temperature ranging between 175 and 220°C.

27. The process according to any one of claims 1 to 8, wherein the esterification is conducted by distillation of water azeotrope until the formation of water ceases.

28. The process according to any one of claims 1 to 8, wherein the catalyst is used in an amount of 50 to 5000 ppm by weight of the reaction mixture.

29. The process according to claim 28, wherein the catalyst is used in an amount of 200 to 1000 ppm by weight of the reaction mixture.

30. The process according to claim 29, wherein the catalyst is used in an amount of 500 to 700 ppm by weight of the reaction mixture.

31. The process according to any one of claims 1 to 8, wherein the ester is di(2-ethylhexyl)phthalate.

32. The process according to any one of claims 1 to 8, wherein the ester is di(2-ethylhexyl)terephthalate.

33. The process according to any one of claims 1 to 8, wherein the ester is 2-ethylhexyl-acrylate.

34. The process according to any one of claims 1 to 8, wherein the ester is triethylene glycol bis (2-ethylhexanoate).

## Patentansprüche

1. Verfahren zur Herstellung von Carbonsäureestern, umfassend die Veresterung einer Carbonsäure mit einem Alkohol in Gegenwart eines titanhaltigen Katalysators, der aus Verbindungen einer allgemeinen Formel ausgewählt ist:
Tiₙ(OR)ₓ(OR')ₓO_{y},
wobei
n für eine Ganzzahl von 1 bis 4 steht;
y für eine Ganzzahl von 0 bis 6 steht;
x gleich oder verschieden sein kann und für eine Ganzzahl von 2 bis 8 steht;
R ein lineares oder verzweigtes C₁-C₁₈Alkyl, C₃-C₁₈Cycloalkyl ist;
R' Aryl ist, das gegebenenfalls einen Elektronendonatorsubstituenten umfasst;
oder eine Mischung davon ist,
mit der Maßgabe, dass
falls n für 1 steht, dann steht x für 2, und y steht für 0; und,
falls n>1, dann umfassen die Verbindungen mindestens zwei Alkoxygruppen und zwei Aryloxygruppen.

2. Verfahren nach Anspruch 1, wobei R' Phenyl ist, das gegebenenfalls einen Elektronendonatorsubstituenten umfasst.

3. Verfahren nach Anspruch 2, wobei der Elektronendonatorsubstituent ein C₁-C₆Alkyl-, Aryl-, C₁-C₆Alkoxy-, C₁-C₆Dialkylamino- oder C₁-C₆Alkylthiogruppe ist.

4. Verfahren nach Anspruch 3, wobei der Alkylteil im Elektronendonatorsubstituenten Methyl, Ethyl, n-Propyl, Isopropyl, n-Butyl, *sec*-Butyl, Isobutyl, *tert-*Butyl, n-Pentyl, *sec*-Pentyl, Isopentyl, *tert*-Pentyl*,* 2,2-Dimethylpropyl, n-Hexyl, Isohexyl, 2,2-Dimethylbutyl, *sec*-Hexyl oder *tert*-Hexyl ist.

5. Verfahren nach Anspruch 3, wobei das Aryl Phenyl ist.

6. Verfahren nach Anspruch 1, wobei R C₁-C₆Alkyl ist.

7. Verfahren nach Anspruch 6, wobei R Methyl, Ethyl, n-Propyl, Isopropyl, n-butyl, *sec*-Butyl, Isobutyl, *tert*-Butyl, n-Pentyl, *sec*-Pentyl, Isopentyl, *tert*-Pentyl, 2,2-Dimethylpropyl, n-Hexyl, Isohexyl, 2,2-Dimethylbutyl, *sec*-Hexyl oder *tert*-Hexyl ist.

8. Verfahren nach Anspruch 1, wobei x gleich ist und für eine Ganzzahl von 2 bis 5 steht.

9. Verbindung nach Anspruch 1, wobei der Katalysator eine Verbindung der allgemeinen Formel (I) oder (II) ist: 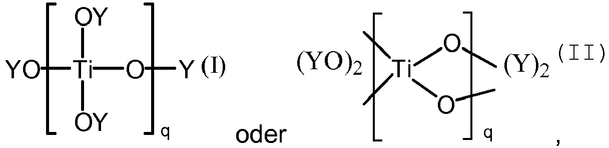 wobei
q für eine Ganzzahl von 1 bis 4 steht;
Y unabhängig R oder R'; wobei R oder R' wie in Anspruch 1 definiert ist, oder eine Mischung davon ist;
mit der Maßgabe, dass die Verbindungen der allgemeinen Formel (I) und (II) mindestens zwei Alkyloxy- und zwei Aryloxygruppen umfassen.

10. Verfahren nach einem der Ansprüche 1 bis 8, wobei der Katalysator eine Verbindung ist, die ausgewählt ist aus:

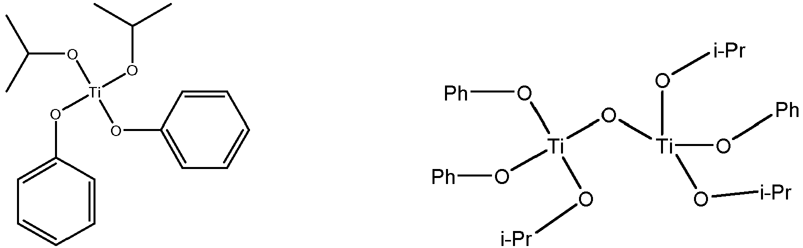

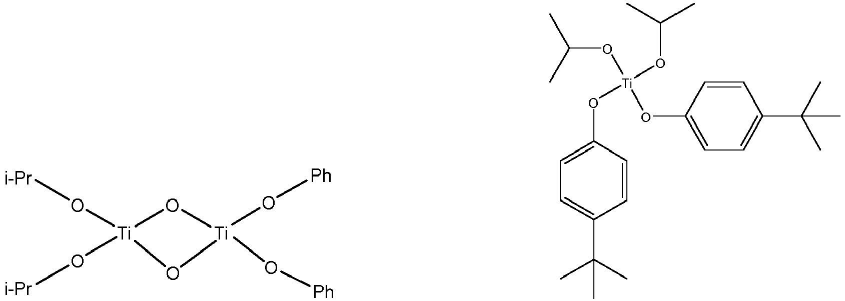

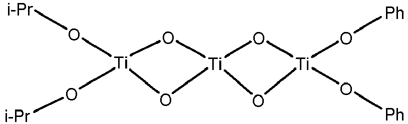

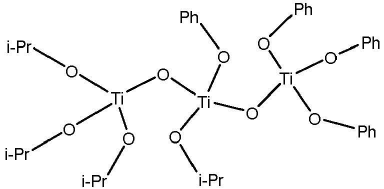

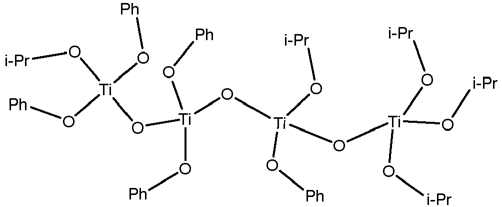

11. Verfahren nach Anspruch 9, wobei der Katalysator Diisopropoxydiphenoxytitan ist.

12. Verfahren nach Anspruch 9, wobei der Katalysator Diisopropoxy- Di(p-*tert-*Butyl)-Phenoxytitan ist.

13. Verfahren nach einem der Ansprüche 1 bis 8, wobei die Carbonsäure eine gesättigte oder ungesättigte mono- oder polybasische Carbonsäure ist, welche eine lineare, verzweigte oder zyklische Struktur aufweist.

14. Verfahren nach Anspruch 12, wobei die Carbonsäure eine aromatische oder aliphatische Carbonsäure ist.

15. Verfahren nach Anspruch 13, wobei die Carbonsäure 3 bis 18 Kohlenstoffatome umfasst.

16. Verfahren nach Anspruch 14, wobei die Carbonsäure 2-Ethylhexan-, Terephthal-, Isophthal-, Phthal-, Benzoe-, Acryl-, Metacryl-, Stearin-, Adipin-, Bernstein-, Sebacin-, Croton-, Zimt-, Linol-, Olein-, Linolen-, Malein-, Fumar-Palmitin-, Laurin- oder Palmitoleinsäure ist.

17. Verfahren nach einem der Ansprüche 1 bis 8, wobei der Alkohol ein monohydrischer oder polyhydrischer Alkohol ist, welcher eine lineare oder verzweigte Struktur aufweist und gegebenenfalls ein oder mehrere Heteroatome, ausgewählt aus O, S oder N, umfasst.

18. Verfahren nach Anspruch 17, wobei der Alkohol 1 bis 18 Kohlenstoffatome umfasst.

19. Verfahren nach Anspruch 18, wobei der Alkohol 2 bis 8 Kohlenstoffatome umfasst.

20. Verfahren nach Anspruch 19, wobei der Alkohol n-Butanol, *sec*-Butanol, Isobutanol, n-Amylalkohol, *sec*-Amylalkohol, Isoamylalkohol, n-Octanol, 2-Ethylhexanol, Monoethylenglycol, Diethylenglycol, Triethylenglycol oder Tetraethylenglycol ist.

21. Verfahren nach einem der Ansprüche 1 bis 8, wobei die Veresterung bei einem Molverhältnis von Carbon-/Hydroxylgruppe von 5:1 bis 1:5 durchgeführt wird, wobei ein Reagenz, das eine azeotrope Mischung mit Wasser bildet, in Anspruch genommen wird.

22. Verfahren nach Anspruch 21, wobei die Veresterung beim Molverhältnis von Carbon-/Hydroxylgruppe von 2:1 bis 1:2 durchgeführt wird.

23. Verfahren nach Anspruch 22, wobei die Veresterung beim Molverhältnis von Carbon-/Hydroxylgruppe von 1,3:1 bis 1:1,3 durchgeführt wird.

24. Verfahren nach einem der Ansprüche 1 bis 8, wobei die Veresterung bei einer Temperatur im Bereich zwischen 100 und 300 °C und unter Druck durchgeführt wird, wodurch das Reaktionsgemisch bei der Temperatur zum Sieden gebracht wird.

25. Verfahren nach Anspruch 24, wobei die Veresterung bei einer Temperatur im Bereich zwischen 160 und 250 °C durchgeführt wird.

26. Verfahren nach Anspruch 25, wobei die Veresterung bei einer Temperatur im Bereich zwischen 175 und 220 °C durchgeführt wird.

27. Verfahren nach einem der Ansprüche 1 bis 8, wobei die Veresterung durch Destillation von Wasserazeotrop bis zur Beendigung der Wasserbildung durchgeführt wird.

28. Verfahren nach einem der Ansprüche 1 bis 8, wobei der Katalysator in einer Menge von 50 bis 5000 Gew.-ppm des Reaktionsgemisches verwendet wird.

29. Verfahren nach Anspruch 28, wobei der Katalysator in einer Menge von 200 bis 1000 Gew.-ppm des Reaktionsgemisches verwendet wird.

30. Verfahren nach Anspruch 29, wobei der Katalysator in einer Menge von 500 bis 700 Gew.-ppm des Reaktionsgemisches verwendet wird.

31. Verfahren nach einem der Ansprüche 1 bis 8, wobei der Ester Di(2-Ethylhexyl)-Phthalat ist.

32. Verfahren nach einem der Ansprüche 1 bis 8, wobei der Ester Di(2-Ethylhexyl)-Terephthalat ist.

33. Verfahren nach einem der Ansprüche 1 bis 8, wobei der Ester 2-Ethylhexylacrylat ist.

34. Verfahren nach einem der Ansprüche 1 bis 8, wobei der Ester Triethylenglycol- Bis(2-Ethylhexanoat) ist.

## Revendications

1. Procédé de préparation d'esters d'acide carboxylique, comprenant l'estérification d'un acide carboxylique avec un alcool en présence d'un catalyseur contenant du titane choisi parmi les composés de formule générale :
Tiₙ(OR)ₓ(OR')ₓO_{y}
dans laquelle
n est un nombre entier de 1 à 4 ;
y est un nombre entier de 0 à 6 ;
x peut être identique ou différent et est un entier de 2 à 8 ;
R est un alkyle en C₁-C₁₈ linéaire ou ramifié, cycloalkyle en C₃-C₁₈ ; R' est un aryle comprenant éventuellement un substituant donneur d'électrons ;
ou un mélange de ceux-ci,
à condition que
si n est 1, alors x est 2 et y est 0 ; et,
si n>1, alors les composés comprennent au moins deux groupes alcoxy et deux groupes aryloxy.

2. Procédé selon la revendication 1, dans lequel R' est un phényle comprenant éventuellement un substituant donneur d'électrons.

3. Procédé selon la revendication 2, dans lequel le substituant donneur d'électrons est un groupe d'alkyle en C₁-C₆, d'aryle, d'alcoxy en C₁-C₆, de dialkylamino en C₁-C₆ ou d'alkylthio en C₁-C₆.

4. Procédé selon la revendication 3, dans lequel la partie alkyle dans le substituant donneur d'électrons est méthyle, éthyle, n-propyle, isopropyle, n-butyle, *sec*-butyle, isobutyle, *tert*-butyle, n-pentyle, *sec*-pentyle, isopentyle, *tert*-pentyle, 2,2-diméthylpropyle, n-hexyle, isohexyle, 2,2-diméthylbutyle, *sec*-hexyle ou *tert*-hexyle.

5. Procédé selon la revendication 3, dans lequel l'aryle est un phényle.

6. Procédé selon la revendication 1, dans lequel R est alkyle en C₁-C₆.

7. Procédé selon la revendication 6, dans lequel R est méthyle, éthyle, n-propyle, isopropyle, n-butyle, *sec*-butyle, isobutyle, *tert*-butyle, n-pentyle, *sec-*pentyle, isopentyle, *tert*-pentyle*,* 2,2-diméthylpropyle, n-hexyle, isohexyle, 2,2-diméthylbutyle, *sec*-hexyle ou *tert*-hexyle.

8. Procédé selon la revendication 1, dans lequel x est identique et est un nombre entier de 2 à 5.

9. Procédé selon la revendication 1, dans lequel le catalyseur est un composé de formule générale (I) ou (II) : 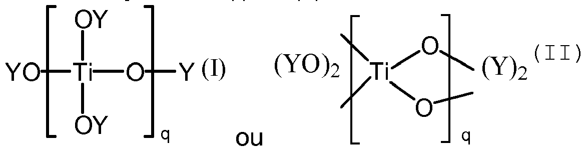 dans lequel
q est un nombre entier de 1 à 4 ;
Y est indépendamment R ou R' ; dans laquelle R ou R' est tel que défini dans la revendication 1,
ou un mélange de ceux-ci ;
à condition que les composés de formule générale (I) et (II) comprennent au moins deux groupes alcoxy et deux groupes aryloxy.

10. Procédé selon l'une quelconque des revendications 1 à 8, dans lequel le catalyseur est un composé choisi parmi :

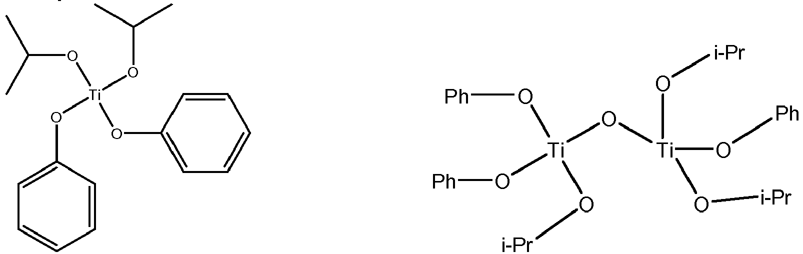

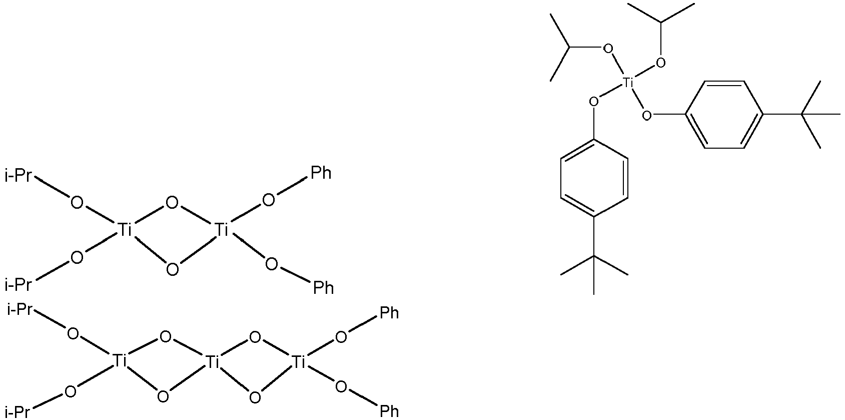

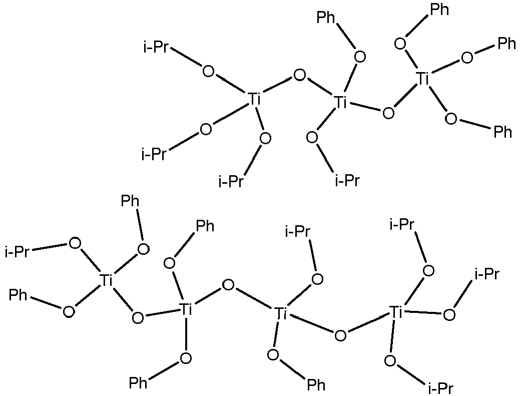

11. Procédé selon la revendication 9, dans lequel le catalyseur est le diisopropoxy diphénoxy titane.

12. Procédé selon la revendication 9, dans lequel le catalyseur est le diisopropoxy di(p-*tert*-butyl)phénoxy titane.

13. Procédé selon l'une quelconque des revendications 1 à 8, dans lequel l'acide carboxylique est un acide carboxylique mono- ou polybasique saturé ou insaturé ayant une structure linéaire, ramifiée ou cyclique.

14. Procédé selon la revendication 12, dans lequel l'acide carboxylique est un acide carboxylique aromatique ou aliphatique.

15. Procédé selon la revendication 13, dans lequel l'acide carboxylique comprend 3 à 18 atomes de carbone.

16. Procédé selon la revendication 14, dans lequel l'acide carboxylique est 2-éthylhexanoïque, téréphtalique, isophtalique, phtalique, benzoïque, acrylique, méthacrylique, stéarique, adipique, succinique, sébacique, crotonique, cinnamonique, linolique, oléinique, linolénique, maléique, fumarique, palmitique, acide laurique ou acide palmitoléique.

17. Procédé selon l'une quelconque des revendications 1 à 8, dans lequel l'alcool est un alcool monohydrique ou polyhydrique ayant une structure linéaire ou ramifiée et comprenant éventuellement un ou plusieurs hétéroatomes choisis parmi O, S ou N

18. Procédé selon la revendication 17, dans lequel l'alcool comprend 1 à 18 atomes de carbone.

19. Procédé selon la revendication 18, dans lequel l'alcool comprend 2 à 8 atomes de carbone.

20. Procédé selon la revendication 19, dans lequel l'alcool est n-butanole, sec-butanol, isobutanole, alcool n-amylique, alcool *sec*-amylique, alcool isoamylique, n-octanole, 2-éthylhexanole, monoéthylèneglycole, diéthylèneglycole, triéthylèneglycole ou tétraéthylèneglycole.

21. Procédé selon l'une quelconque des revendications 1 à 8, dans lequel l'estérification est effectuée dans un rapport molaire groupe carboxylique / hydroxyle de 5:1 à 1:5, dans lequel un réactif formant un mélange azéotropique avec de l'eau est prélevé.

22. Procédé selon la revendication 21, dans lequel l'estérification est effectuée dans un rapport molaire du groupe carboxylique / hydroxyle de 2:1 à 1:2.

23. Procédé selon la revendication 22, dans lequel l'estérification est effectuée dans un rapport molaire du groupe carboxylique / hydroxyle de 1,3:1 à 1:1,3.

24. Procédé selon l'une quelconque des revendications 1 à 8, dans lequel l'estérification est effectuée à une température comprise entre 100 et 300°C et sous pression, en faisant bouillir le mélange réactionnel à ladite température.

25. Procédé selon la revendication 24, dans lequel l'estérification est effectuée à une température comprise entre 160 et 250°C.

26. Procédé selon la revendication 25, dans lequel l'estérification est effectuée à une température comprise entre 175 et 220°C.

27. Procédé selon l'une quelconque des revendications 1 à 8, dans lequel l'estérification est effectuée par distillation de l'eau azéotrope jusqu'à ce que la formation d'eau cesse.

28. Procédé selon l'une quelconque des revendications 1 à 8, dans lequel le catalyseur est utilisé en une quantité de 50 à 5000 ppm en poids du mélange réactionnel.

29. Procédé selon la revendication 28, dans lequel le catalyseur est utilisé en une quantité de 200 à 1000 ppm en poids du mélange réactionnel.

30. Procédé selon la revendication 29, dans lequel le catalyseur est utilisé en une quantité de 500 à 700 ppm en poids du mélange réactionnel.

31. Procédé selon l'une quelconque des revendications 1 à 8, dans lequel l'ester est le phtalate de di(2-éthylhexyle).

32. Procédé selon l'une quelconque des revendications 1 à 8, dans lequel l'ester est le téréphtalate de di(2-éthylhexyle).

33. Procédé selon l'une quelconque des revendications 1 à 8, dans lequel l'ester est 2-éthylhexyl-acrylate.

34. Procédé selon l'une quelconque des revendications 1 à 8, dans lequel l'ester est bis(2-éthylhexanoate) de triéthylèneglycol.
